# EUROPEAN PATENT APPLICATION

(11) **EP 4 851 986 A2**
(43) Date of publication of application: **09.12.2026**
(21) Application number: 26193466.5
(22) Date of filing: 02.09.2021
(51) Int. Cl.: G10H 1/00

(54) **SYSTEM AND METHOD FOR REPRESENTING A BIOLOGICAL PROCESS VIA NON-SPEECH AUDIO**

(30) Priority: 08.09.2020 US 202063075336 P
(62) Document number(s) of the earlier application(s) in accordance with Art. 76 EPC: 21866210.4 / 4 211 459
(71) Applicant: AIVF Ltd., 6971003 Tel-Aviv (IL)
(72) Inventor: GILBOA, Daniella, 4335719 RaAnana (IL); WEINBERG, Gil, Atlanta, Georgia, 30350 (US)
(74) Representative: Pearl Cohen UK

(57) **Abstract**

A method of representing a biological process via non-speech audio and system for carrying out same are provided. The method is effected by extracting a sequence of time-related biological events from the biological process and transforming the sequence of time-related biological events into rhythm and/or melody representative of the sequence of time-related biological events thereby representing the biological process via non-speech audio.

## Description

### RELATED APPLICATION/S

This application claims the benefit of priority of U.S. Provisional Patent Application No. 63/075,336 filed on September 8, 2020, the contents of which are incorporated herein by reference in their entirety.

### FIELD AND BACKGROUND OF THE INVENTION

The present invention relates to a system and method for representing a biological process via non-speech audio (e.g., music). Embodiments of the present invention relate to sonification of an embryonic development video to generate a user audible rhythm and/or melody that can be used to identify/verify embryos suitable for implantation.

In Vitro Fertilization (IVF) has been used to treat infertility problems successfully since 1978. Despite on-going research it is still a complicated procedure with a success rate of only 20% using the best available resources.

IVF is an expensive procedure that is psychologically traumatic for a patient and as such, identifying recipients for whom IVF is unlikely to be successful prior to treatment, or embryos most suitable for implantation can reduce costs associated with an IVF procedure and the discomfort such a procedure causes the patient.

The embryo selection step of an IVF procedure is crucial to implantation success. Selection is typically carried out manually via microscopic screening of embryos throughout their development cycle. More recently, time-lapse video microscopy has enabled prolonged time lapse capture of microscopy images that can be analyzed automatically or manually to determine suitability for implantation.

While video-based screening approaches increase the likelihood of successful implantation especially when manually carried out by experienced technicians, additional screening tools are needed in order to increase the likelihood of successful implantation.

While reducing the present invention to practice, the present inventors have devised an approach for screening biological processes such as embryo development. The present approach can supplement manual or automated video-based screening and provides a technician with an additional layer of information that can be used in a decision making process.

### SUMMARY OF THE INVENTION

According to one aspect of the present invention there is provided a method of representing a biological process via non-speech audio comprising: extracting a sequence of time-related biological events from the biological process; and transforming the sequence of time-related biological events into musical sonification representative of the sequence of time-related biological events thereby representing the biological process via non-speech audio.

According to embodiments of the present invention, the sequence of time-related biological events is extracted from a video or time lapse capture of the biological process.

According to embodiments of the present invention, the biological process is embryonic development.

According to embodiments of the present invention, the sequence of time-related biological events includes cellular division, growth and/or differentiation.

According to embodiments of the present invention, the sequence of time-related biological events includes changes to a subcellular structure.

According to embodiments of the present invention, the subcellular structure is a nucleus, a pronucleus, cytoplasm or a cytoskeleton.

According to embodiments of the present invention, the rhythm is percussion rhythm.

According to embodiments of the present invention, the method further comprises combining the rhythm and/or melody representative of the time-related biological events to the video or time lapse capture of the biological process.

According to embodiments of the present invention, the rhythm and/or melody representative of the time-related biological events of a normal biological process differs from that of an abnormal biological process.

According to embodiments of the present invention, the rhythm and/or melody representative of the time-related biological events of a normal biological process is more rhythmic and/or melodic than that of an abnormal biological process.

According to embodiments of the present invention, the sequence of time-related biological events is extracted from a video or time lapse capture of the biological process using image recognition software.

According to embodiments of the present invention, the rhythm and/or the melody is analyzed for changes over time.

According to embodiments of the present invention, the rhythm and/or the melody is analyzed using a signal-processing algorithm.

According to embodiments of the present invention, the signal-processing algorithm extracts human-perceivable and quantifiable high-level musical information.

According to embodiments of the present invention, the signal-processing algorithm extracts a rhythmic periodicity of the rhythm and/or melody.

According to embodiments of the present invention, the signal-processing algorithm measures a self-similarity of the rhythm and/or melody.

According to another aspect of the present invention there is provided a system for representing a biological process via non-speech audio comprising a computational unit configured for extracting a sequence of time-related biological events from the biological process; and transforming the sequence of time-related biological events into rhythm and/or melody representative of the time-related biological events thereby representing the biological process via non-speech audio.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

Implementation of the method and system of the present invention involves performing or completing selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

### BRIEF DESCRIPTION OF THE SEVERAL VIEWS OF THE DRAWING(S)

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 schematically illustrates one embodiment of the present system.
FIG. 2 is an image showing mapping of cell division events (t events) for the major scale.
FIG. 3 illustrates a self-similarity matrix analysis of musical structure.
FIGs. 4A-B, 5A-B, 6A-B illustrate rhythm/melody audio tracks aligned with several images representing a portion of a time lapse video depicting development of a successfully implanted (Figure 4A, 5A, 6A) and a non-successfully implanted (Figure 4B, 5B, 6B) embryo.

### DESCRIPTION OF SPECIFIC EMBODIMENTS OF THE INVENTION

The present invention is of a system which can be used to represent a biological process via rhythm and/or melody. Specifically, the present invention can be used to represent embryonic development in a manner which enables a technician to acoustically detect, which embryos are more likely to be successfully implanted.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Embryo selection in IVF procedures is a crucial step to implantation success. Although advancements have been made in image capture of developing embryos and automatic analysis of captured images, the success of a procedure still relies on the skill and experience of the technician. In a previously filed application, the present inventors devised an automated system for identifying predictors of successful IVF implantation from images (e.g., of time lapse videos) captured from successfully and non-successfully -implanted embryos.

Addressing the need for additional tools, the present inventors devised a system that can provide another layer of information that harnesses the acoustic assessment of a user or automated system to substantially enhance an embryo screening procedure prior to implantation.

Thus, according to one aspect of the present invention there is provided a method of representing a biological process via non-speech audio.

The method is carried out by extracting a sequence of time-related biological events from the biological process and transforming this sequence into a representative rhythm and/or melody (also termed herein as "musical sonification").

The biological process can be any process that includes a sequence of biological events over time. Examples of such processes include, but are not limited to, disease progression, tumor growth, organ development, brain activity, heartbeat patterns, blood pressure changes, respiratory system and embryonic development. The biological events can be any macro or micro changes to a subcellular structure, a cell or a collection of cells (e.g., tissue/organ) that can be observed. Examples of biological events include, but are not limited to, cell division, cell growth, tumor growth or shrinkage, tissue/organ growth, migration or changes in subcellular structures such as nuclei, pronuclei, cytoplasm, cytoskeleton, endoplasmic reticulum and the like. These sequences can be monitored in real time or, if their pace is slower or faster, using time compression or expansion of the series of events.

The time-related biological events can be extracted from a video (e.g., time lapse video capture of a microscopic scan) of the biological process via, for example, an image recognition software. The image recognition software can automatically identify and tag the biological events with a time stamp and an identifier. For example, in the case of cell division, image analysis implemented by means such as a convolutional neural network can identify a cell division event and tag it with a time stamp and a 'cell division' identifier. Such an event can then be used to generate a specific portion of the rhythm, melody, harmony and / or timbre as is further described hereinbelow.

Once the entire biological process (e.g., IVF embryo development from insemination to transfer into the womb) is sonified, musical sonification can be used to assess the nature of the biological process. For example, in the case of development (e.g., embryo), the audio track can be used to assess whether the development is normal or not. The audio track can also be aligned to, and combined with the video or time lapse capture of the biological process to provide an additional layer of information to a technician analyzing the video.

As is further described hereunder, musical sonification representative of time-related biological events of a normal biological process differs from that of an abnormal biological process.

For example, a normal biological process can be perceived as more rhythmic than that of an abnormal biological process. More rhythmic in this context can mean that the rhythm is more regular; e.g., that it consists of rhythmic events that mainly fall on a beat. Less rhythmic patterns consist of more events that occur between beats also known as syncopation. Less rhythmic patterns might also consist of such irregularly spaced events that no beat is detectable at all. Complex processes such as cell division may result in multiple concurrent patterns, where any part of the overall sequence may or may not be rhythmic. For example, if some cells divide normally and some abnormally, the musical equivalent could be a beat with jarring noise superimposed.

As is mentioned hereinabove, the musical sonification can be analyzed by a user (e.g., technician) or by an automated system. In the case of the former, the various constituent aspects of the musical output can be designed to intuitively convey "correctness" of a given input. The musical aspects used can include multiple musical aspects such as rhythm, melody, timbre, and harmony. Rhythm, as described above, can convey correctness intuitively via the level of syncopation; a more syncopated (or less regular) rhythm will sound more chaotic and thus intuitively give the impression to the listener that the input may be less than ideal (less correct).

Melody can be used to convey correctness by relying on common patterns found throughout western tonality. For example, individuals familiar with western music will intuitively hear melodies that strongly suggest a diatonic major key as happy, positive, or good and will alternatively hear melodies that do not suggest any diatonic tonal center as strange, uncomfortable, or unpredictable. This way, one can vary how strongly the melodies suggest a major diatonic center to convey to the listener how fit a given input is.

Timbre is a term that can convey the tone, or "color" of the musical sounds. For example, one might describe a trumpet as having a harsher timbre than a French horn. In the present system, timbre is varied by introducing more noise and distortion to the output in order to produce a less pleasant tone. Sounds with more noisiness and distortions will be intuitively heard as less compelling by the listener.

Harmony is used in conjunction with the melodic aspects of the sonification to convey "correctness". More successful inputs will produce output that contains a drone tone that enforces the diatonic center suggested by the melodic content, whereas less successful examples will contain drone tones that are heavily distorted and are less related to a tonal musical center. When the drone tone is unrelated to the melodic content, the output will be heard as dissonant to listeners with experience with western music, and can convey a strong sense of incorrectness.

In the case of automated analysis, the musical sonification is generated based on a signal-processing algorithm that extracts human-perceivable and quantifiable high-level musical information (e.g., rhythmic periodicity of the rhythm and/or melody) and can measure self-similarity of the rhythm and/or melody.

Self-similarity is used to detect patterns within a given input. So, a more rhythmic pattern for instance, where events occur at more regularly-spaced intervals, will be more self-similar than a pattern with more varied or chaotic spacing. This automated approach can be used to examine large amounts of sonification data without the need to individually listen to and evaluate each example.

The human auditory cortex is highly tuned to detect patterns in temporal input. For example, the human brain can notice time difference between two audio stimuli that are separated by as little as 10 milliseconds. Such subtle time differences cannot be identified through visualization. Therefore, musical sonification bears can better represent patterns in time based events (such as embryonic cell division sequences) in comparison to visual based representation.

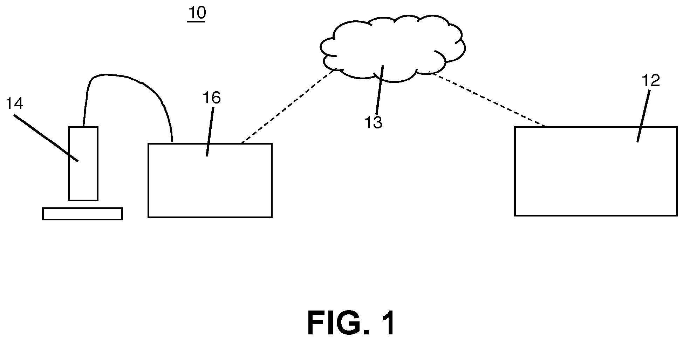

Referring now to the drawings, Figure 1 illustrates one embodiment of a system for representing a biological process via non-speech audio (also referred to herein as sonification) which is referred to herein as system 10.

System 10 is described hereunder in the context of embryo development, it will be understood however, that system 10 can also be used to assess alternative biological processes.

System 10 includes a computing platform 16 configured for obtaining and optionally storing a sequence of time-stamped images tracking development of a pre-implantation embryo. System 10 can be in communication (e.g. through cloud 13 or wire connection) with a data storage device 12 storing the images (time lapse) retrieved from an image capture device 14 (e.g., microscope with camera) or it can be in direct communication with image capture device 14 through a wired or wireless (e.g., cloud 13) connection.

The time-stamped images can be derived from a video capture, a time lapse capture or a still capture at various time intervals. For example, the images can be derived from stored image data that includes time lapse/video/still images obtained from an embryo.

In any case, the time-stamped images represent a time period of ~150 hours in embryo development (the time from fertilization to 6 days post fertilization) from 0 hours until ~150 hours and can be spaced apart at 1 second to 20 minute intervals.

The following represents typical time points of biological events in development of a fertilized oocyte to implantation-ready embryo that can be included in the time stamped sequences of images utilized by the present invention for sonification of embryonic development.

t0: The time at which insemination occurs in conventional IVF. For ICSI/IMSI, where the time-lapse monitoring system and practice allows, the time of the sperm injection may be recorded, per oocyte but otherwise, it is the mid-time point from when injection begins and ends for that patient's cohort of oocytes. This time point can be used as a start time.

tPB2: The time at which the second polar body (PB2) is extruded. This is annotated at the first frame in which PB2 appears completely detached from the oolemma. The extrusion of the second polar body can be obscured depending on the position of the oocyte in the well or by cumulus cells in routine IVF insemination.

tPN: The time at which fertilization status is confirmed. It is recommended to annotate fertilization immediately before fading of pronuclei (tPNf) hence coinciding to tZ (time of pronuclear scoring), since no further observational dynamic changes are expected to occur. Appearance of individual pronuclei may be further annotated as tPNna ('n' for individual pronuclei in the order of appearance: 'a'): e.g. tPN1a, tPN2a, tPN3a the initial time at which the first, second, third, etc. pronuclei become visible.

tPNf: The time when both (or the last) PN disappear. This annotation is made at the first frame whereby the embryo is still at the 1-cell stage but pronuclei can no longer be visualized. Pronuclear fading may be further recorded according to individual pronuclei, tPN1f, tPN2f, etc. to denote the time at which the first, second or additional pronuclei fade (i.e. similar to annotation of their appearances).

tZ: The time of time-lapse PN assessment. PN are dynamic structures; they move and their morphology can change between tPNa and tPNf (Azzarello et al., 2012). It has recently been reported that the movement of the pronuclei within the cytoplasm and fading of nuclear membranes may be indicative of subsequent blastocyst development potential and hence a novel parameter providing an early indication of the embryo's developmental potential (Wirka et al., 2013). Changes in pronuclear appearance and position may coincide with movement of the nucleolar precursor bodies (NPBs) inside pronuclei, allowing differential PN scoring to be deduced. The time-lapse user group recommends annotation of PN scoring, if required, at the last frame before the pronuclei disappear (i.e. tPNf) because the alteration in pronuclear morphology has been completed.

t2: The time of the first cell cleavage, or mitosis. t2 is the first frame at which the two blastomeres are completely separated by individual cell membranes.

t3: The first observation of three discrete cells. The three cells stage marks initiation of the second round of cleavage.

tn: The first time these numbers of discrete cells are observed (until compaction of blastomeres prevents visualization of individual cells).

tSC: The first frame in which evidence of compaction is present; the initial frame that any (two) cells start to compact is observed.

tMf/p: This marks the end of the compaction process; when observable compaction is complete. The morula may be fully or partially compacted, where f is full and p is partial; the morula has excluded material. The degree and time of compaction has been reported to be associated with blastocyst formation and quality (Ivec et al., Fertility and sterility, Volume 96, Issue 6, December 2011, Pages 1473-1478.e2 2011).

Any of the above biological events can be used in creating the audio track (rhythm/melody) of the present invention.

Once an audio track of embryonic development is generated, the present system can play the track to a user along with, or separately from, the video. A listener will then be able to play the audio, and hear the various musical aspects described above (melody, rhythm, timbre, and harmony), to be able to make an intuitive assessment of the output.

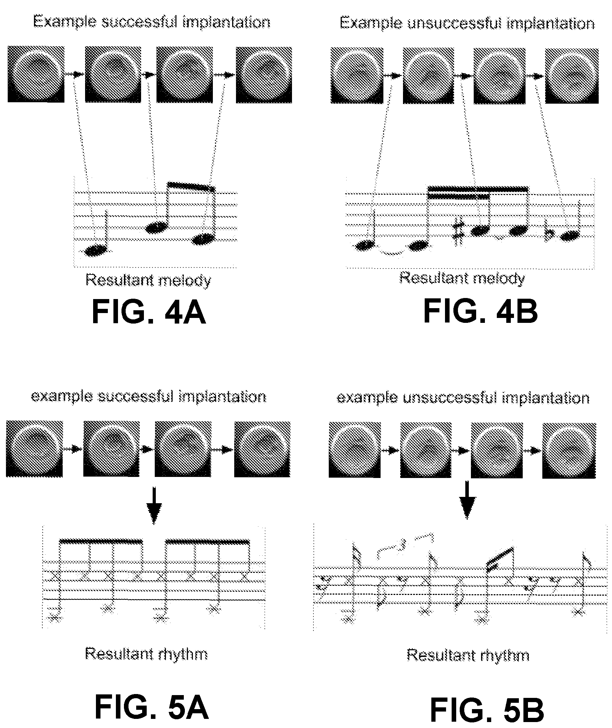

Figures 4A-B, 5A-B and 6A-B illustrate examples of musical sonification of a successfully implantation (Figure 4A, 5A, 6A) and an unsuccessful implantation (Figure 4B, 5B, 6B). The images on top represent stages in embryo development: the first, second and third mitosis events (division of a cell into two), the images on the bottom represent sonification. After fertilization, the egg goes through a process which ends in a blastocyst, a mass of cells ready for implantation in the womb. Mitosis exhibits a temporal pattern specific to the embryo so that, for example, if the first cell divides early - relative to the average of embryos - then the second and third cells are expected to also divide early. Deviations from the expected pattern, as is shown in Figures 4B, 5B and 6B correlate with poor embryo outcomes.

The bottom panels of Figures 4A-6B illustrate different musical elements of the sonification that are generated in accordance with whether division is of a successfully implanted embryo or one that was not successfully implanted. Each of these figures shows three divisions of the candidate embryo (t2, t3, t4), which are represented as the three arrows between the four images of the embryo at the upper portion of each figure. Figure 4A shows a resultant melody for a successfully implanted embryo. Three division events (t2, t3, t4) that are connected with thin lines are illustrated so as to show how these division events directly correspond with the generated melodic events (each division event directly triggers a note to play). The staff in this example is treble clef, but this was left out so as to not overcrowd the diagram. The notes being generated in this example strongly suggest a diatonic major tonality, since the three notes spell out a C major chord. In western music, a major chord is often associated with positivity in general, so its use here corresponds nicely with the success of this embryo.

Figure 4B is an example of an unsuccessfully implanted embryo and the resulting melody generated by the present invention. Unlike the melody in 4A, the melody in 4B does not strongly suggest a diatonic tonality. The first two notes in the 4B melody are a distance apart that is known as a "tritone". In western music, a tritone is often considered a harsh interval that is most often used to indicate uneasiness of some sort, so the presence of this interval and lack of implied tonality in this melody reinforce to the listener that this embryo is not a successful one.

Figure 5A shows a rhythm that is generated for an embryo that was successfully implanted. The rhythm generated here is essentially a basic rock and roll beat. In this rhythm, each downbeat is stressed by a kick drum hit- represented as the low a in this notation (these examples, like examples 4A and 4B are also written in treble clef)- with the weak beats or upbeats being stressed by a snare hit (middle c), so each drum hit occurs directly on a beat. There is a straight eighth note high-hat cymbal pattern happening along with these drum hits that helps give the rhythm a strong and apparent sense of beat. The regularity of this rhythm should convey a sense of "expectedness" to the listener; the strong sense of beat and typical beat-stressing pattern of the rhythm correspond well with the example embryo's success.

Figure 5B is an unsuccessfully implanted embryo and its resultant rhythm, unlike the rhythm of Figure 5A, most drum beats do not occur on the beat. When rhythms have events that occur off the beats, this is known as syncopation. Syncopation does not necessarily convey a negative feeling to the user like, for example, an atonal melody, however these rhythms are often heard as more exotic and possibly even strange. Only one drum beat actually aligns with a beat (the second kick drum hit), however this doesn't wind up giving this rhythm any more perceived regularity. Since the rest of the elements are so irregular, a listener may hear this rhythm as being almost, if not entirely, arrhythmic. This irregular and highly syncopated rhythm clearly conveys that this embryo is not successful.

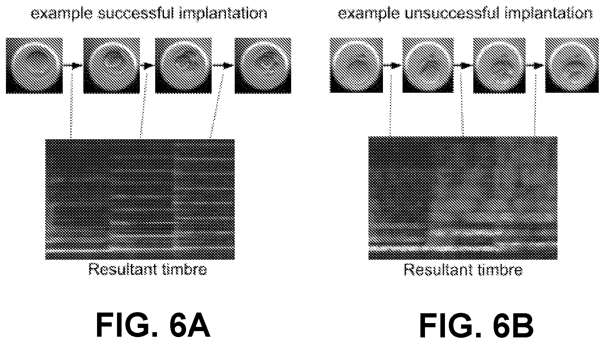

Figures 6A and 6B are spectrograms of a generated melody of the sonication. The spectrograms are used to illustrate how the sound elements of the melody, beyond just its constituent notes, are generated so as to reflect the success or failure of a candidate embryo.

Figure 6A shows the timbral component of a sonification performed for a successfully implanted embryo. A spectrogram is used herein to illustrate this timbral component. A spectrogram shows us the frequency content of a given sound and demonstrates nicely divided parallel lines in the Example of Figure 6A. The parallel lines are segmented into three sections, each of different vertical offset; this simply represents that there are three separate notes played. Much like examples 4A and 4B, direct mapping between the division events (t2, t3, and t4, represented as the three arrows in the upper portion of the figure), to the three separate notes visible in the spectrogram is shown. In this case, importance is placed on how distinct each of the parallel lines in the spectrogram is; this implies that these notes will have a clear pitch. Thus, a successful embryo will have a timbre that is very clear and does not include any noisy sonic components.

Figure 6B shows the spectral (or timbral) component of a resultant sonification of a failed embryo. The same parallel line structure as in figure 6A is shown, but it is clear that the lines are far less distinct; this means that the tones/pitches of this example would be far less clear and would sound a fair amount more noisy than those in Figure 6A. The noisiness and lack of clear tones conveys a more negative impression to the user, since a more noisy sound will typically be perceived as strange or even unpleasant than an equivalent less noisy sound.

As is mentioned hereinabove, the present invention can also be suitable for representing disease progression, tumor growth, organ development, brain activity, heartbeat patterns, blood pressure changes, respiratory system as well as other pathologies, conditions and disorders.

As used herein the term "about" refers to ± 10 %.

Additional objects, advantages, and novel features of the present invention will become apparent to one ordinarily skilled in the art upon examination of the following examples, which are not intended to be limiting.

### EXAMPLE

Reference is now made to the following example, which together with the above descriptions, illustrate the invention in a non-limiting fashion

### Sonification Of Embryonic Development

A system for sonifying time-lapse videos of fertilized embryos was developed and tested. The system enables to explore embryonic development in the sonic domain. This system has three main proposed uses (i) detection of embryos that are more likely to succeed, (ii) provide an aesthetic fingerprint for an embryo for artistic purposes to ameliorate the process mothers go through while undergoing in vitro fertilization and (iii) provide researchers with new tools and techniques found in music and sound research that can be applied to screening of biological processes.

The sonification system utilizes percussion, drum loops, melodies, drones and the like to convert time-stamped biological events present in time lapse videos of embryo development to musical sonification audible by a user. Each video is analyzed by a data analysis system, producing various time-series data points. The data points used in this study are labelled t2, t3, t4, t5, t6, t7, t8, and t9 and correspond to cell division event (t3 for example, is the first frame at which at least 3 cells appear).

The percussion feature of the sonification is the first and most basic feature. Each of the eight t events trigger a unique percussion sample. This generates a unique percussion track for each embryo video. The user is also able to choose one or more percussion sounds to be mapped to each t event. The percussion sounds can include various kick drum, hand drum, and cymbal samples.

The drum loop feature allows for a drum loop to be played in time with the time-lapse video, such that the drum loop repeats seamlessly. This feature is added to add a background rhythmic context upon which the other sonification elements can be stacked. The idea is that this background rhythmic context may help users interpret the sonification in a more rhythmic sense; allowing users to detect subtle time differences that might not be detectable outside of a non-musical context. The other use of the drum loop is to provide an aesthetic backbone for the sonification to increase overall musicality.

The melody feature of the sonification is much like the percussion feature except that the t events are mapped to different notes of a musical scale rather than to different percussion samples. There are two modes that the melody mode operates under, custom scale mode and a fixed scale mode. The fixed scale mode is the more simple of the two modes. In the fixed scale mode, each t event (2 through 9) is mapped to this pattern of scale degrees: 1, 3, 8, 6, 4, 2, 5, 7. The system can have 7 scales to choose from: major, minor, pentatonic, persian, nahawand, bayati, and jiharkah. Nahawand, bayati, and jiharkah are scales taken from the Arabic maqam tradition. The pattern for scale degrees was mainly chosen for the way grouped events form a pleasant implied chord progression when a western diatonic scale (major or minor) is chosen.

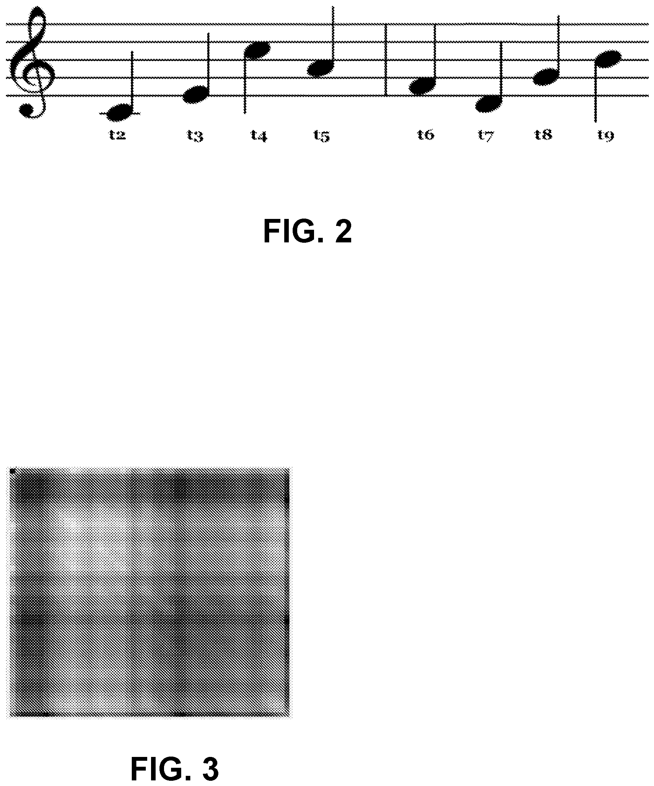

Figure 2 illustrates an example of mapping of t events for the major scale. Here, the implied chord progression is I - ii - V, a very common progression in wester harmony. If the chosen scale does not have at least 7 distinct notes (the pentatonic scale for instance), the scale is continued beyond an octave until there are enough notes to create the scale degree pattern. This means that, this pattern does not create a nice implied chord progression in all cases (ie the pentatonic scale). However, the scale degree pattern also makes sure that two consecutive t events trigger notes that are not consecutive in the desired scale; this will generally avoid dissonant-sounding chords in cases where two t events occur close together. In the more complex custom scale mode, the relative spacing of the t events determine the frequencies of each note in the scale. This means that each embryo will have a unique scale associated with it. The midi pitch of the ith degree of the custom scale is represented by the equation: $Pitch \left(i\right) = 60 + \frac{{t}_{i} - {t}_{2}}{{t}_{9} - {t}_{2}}$

This midi pitch can then be translated into the desired frequency of each note in the custom scale.

The first variation of the drone provides an aesthetic backdrop to the other sonification features and is based on overall visual activity. Both this variant of the drone and the second variant are different from the other sonification features in that they analyze the video directly, whereas the other sonification features rely on higher-level labeled data (the t events). The analysis for this feature is quite primitive and low level. An average pixel difference between two consecutive video frames is determined and fed into a buffer in order to use a running median filter on the previous 11 values to produce a filtered stream of data that corresponds to the amount of movement occurring in the video. This stream of data is then mapped onto various synthesizer parameters in order to produce a droning sound that becomes brighter and more active in its tone as the video becomes more active in terms of movement. This drone is centered on a C note, which was chosen as the common starting note of all of the scales present in the melody feature, making the drone musically complimentary to the rest of the sonification soundscape.

The second variant of the drone, much like the first variant, analyzes the video to capture movement by using the Horn-Schunk technique to calculate optical flow. Optical flow is typically used in video compression algorithms; it attempts to locate only pixels that move from frame to frame and then encode only that pixel movement instead of the whole video frame, thus compressing the video file size. This study did not use optical flow to compress the videos, but rather to determine movement in the videos. The optical flow data was broken into four frames, corresponding to all the pixel movement occurring in each direction (left, right, up, & down). Each of these frames was then averaged and filtering applied in a manner similar to that of the first variant of the drone producing four streams of data rather than just one. The basic or the drone is created by mapping these four data streams onto the operator frequencies of a four-operator fm synthesizer. The four streams of data were then used to spatialize the drone. The spatialization of this drone is the only aspect of the sonification that relies on stereo audio. The basic idea of the mapping for spatialization purposes is to make the left or right channel more or less active based on the amount of optical flow to the left or right. For further modulation of the drone, there is another idea at play in this mapping that requires some description of the input videos.

The videos show cells growing and dividing it is therefore expected that movement in the video would be similar in the four basic directions, since the cells appear as circles in the videos. However, when cells divide, there is less of an expectation that movement will be similar in all directions. In order to represent such asymmetry in movement, the similarity between the four streams of optical flow data was mapped to various parameters of the drone synthesizer to render the drone more distorted and active when similarity is lower. Since this variant of the drone is a constantly changing pitch, it does not necessarily compliment the melody feature but can be made more musical.

### Data Analysis

Data analysis utilized a self-similarity matrix (SSM) to perform structural analysis of the sonification audio assuming that a technique often used in musical structural analysis- the SSM-might be applicable to this domain.

SSM are used for musical structural analysis, the basic idea behind the SSM is to show how similar each point throughout an input audio sample is similar to each other point. An Example of a computed SSMs is shown in Figure 3 in which brighter pixels correspond to higher degrees of similarity.

In order to calculate the SSM, the sonification audio was broken into windows 25ms in length sampled every 100 ms by default. The interface allows these values to be changed by the user. The Mel Frequency Cepstral Coefficients (MFCCs) was then calculated for each of these windows and then each feature vector was grouped with the 9 feature vectors following it, creating groupings of 10 feature vector. These groupings of 10 feature vectors are then compared with each other grouping by summing the pairwise dot products of each group, producing a single similarity score. So, given feature vectors F[1..n], and group size S (10 by default) an (n-S-1) by (n-S-1) matrix is computed where each cell in the matrix - for coordinates x and y where both x and why range between 1 and (n-S-1) - is represented by the equation: $\left|cell\left(x, y\right)\right. = {\sum }_{i = 1}^{S} F \left⌈x+i-1\right⌉ ⋅ F \left⌈y+i-1\right⌉ .$

The dataset includes 2248 time lapse videos, each with corresponding pre-calculated time-series data. One piece of data associated with each embryo video is a flag called "beta_pregnancy_test", which indicates whether an embryo was successfully implanted or not. The dataset was divided based on this flag to 1197 failed embryos and 1051 successful embryos.

To examine if there is a significant difference in overall similarity between the successful embryos and the failed embryos a batch output feature was added to the sonification software. Batch output allows a user to specify a folder containing multiple videos files for which the user wishes to compute SSMs. Once a folder is selected, the system automatically sonifies each file, then it computes the SMM for each sonification. Each SSM is stored as a CSV file, rather than an image, so that data can be precisely saved and reloaded for further analysis. The SSMs were batch calculated for all of the successful and failed embryos. Since the sonifications typically contained some amount of silence at the beginning or end, a threshold to remove points of low similarity at the edges of the SSMs was set. The average similarity for the entire matrix was then calculated and the average similarity score for all of the successful and failed embryos was calculated. The means of these two sets was then compared and the results showed that successful embryos have higher average similarity scores in general than failed embryos. Running a t-test produces a t statistic of 4.56 with a p value of 5.80e⁻⁶.

As is mentioned hereinabove, the data used in sonification includes a sequence of time events labeled t2 through t9, representing the times at which cell divisions occur. Since sonification represents a mapping of these events to particular sounds, embryo division data was used to compute SSMs without relying on the sonification audio. This approach relies on techniques typically used in musical structural analysis, and was directly inspired by the use of sonification. Computing these SSMs was far simpler than computing the SSMs for the sonified audio. In order to compute each SSM, the series of t2 to t8 was used as the basic input. The similarity (simple euclidean distance) of each rotation of the input vercor to all possible rotations was then computed producing an 8X8 matrix. For illustration, the first rotation of the input vector would look like [t3,t4,t5,t6,t7,t8,t2]; notice t2 is now at the end of the vector after the first rotation. Using this technique on the entire dataset enabled assignment of an average similarity value to each embryo. Results showed that as with the previous approach (computing SSMs from the sonification), successful embryos have higher average similarity scores in general than failed embryos. Running a t-test produces a t statistic of -3.55 with a p value of 0.00040. The sign of the t statistic is negative for this experiment since given the way similarity is computed in this case, smaller values imply more similarity. So this result matches the result from the previous experiment.

The present study then tested if these SSMs could be used as a feature to be fed to a machine learning model used to classify whether a candidate embryo was successful or not by using the 8X8 SSMs generated from looking at the event data directly. The study first balanced the dataset so that there are equal numbers of failed embryos and successful embryos. The data was then divided into 1686 examples used to train the model and 562 examples to test the model; binary classification was performed by a support vector machine. Running the model on the test data, resulted in an area under the curve of the region of convergence graph (AUC-ROC) of 0.55.

From statistical analysis, it is clear that the rhythms generated by division events are significantly more self-similar for successful embryos as opposed to failed embryos.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.
The description also includes the subject matter of the following clauses:
Clause 1. A method of representing a biological process via non-speech audio comprising:
   (a) extracting a sequence of time-related biological events from the biological process; and
   (b) transforming said sequence of time-related biological events into sonification representative of said sequence of time-related biological events thereby representing the biological process via non-speech audio.
Clause 2. The method of clause 1, wherein said sequence of time-related biological events is extracted from a video or time lapse capture of the biological process.
Clause 3. The method of clause 1, wherein the biological process is embryonic development.
Clause 4. The method of clause 3, wherein said sequence of time-related biological events includes cellular division, growth and/or differentiation.
Clause 5. The method of clause 3, wherein said sequence of time-related biological events includes changes to a subcellular structure.
Clause 6. The method of clause 5, wherein said subcellular structure is a nucleus, a pronucleus, cytoplasm or a cytoskeleton.
Clause 7. The method of clause 1, wherein said rhythm is percussion rhythm.
Clause 8. The method of clause 2, further comprising combining said rhythm and/or melody representative of said time-related biological events to said video or time lapse capture of the biological process.
Clause 9. The method of clause 1, wherein said rhythm and/or melody representative of said time-related biological events of a normal biological process differs from that of an abnormal biological process.
Clause 10. The method of clause 9, wherein said rhythm and/or melody representative of said time-related biological events of a normal biological process is more rhythmic and/or melodic than that of an abnormal biological process.
Clause 11. The method of clause 2, wherein said sequence of time-related biological events is extracted from a video or time lapse capture of the biological process using image recognition software.
Clause 12. The method of clause 1, wherein said rhythm and/or said melody is analyzed for changes over time.
Clause 13. The method of clause 12, wherein said rhythm and/or said melody is analyzed using a signal-processing algorithm.
Clause 14. The method of clause 13, wherein said signal-processing algorithm extracts human-perceivable and quantifiable high-level musical information.
Clause 15. The method of clause 13, wherein said signal-processing algorithm extracts a rhythmic periodicity of said rhythm and/or melody.
Clause 16. The method of clause 13, wherein said signal-processing algorithm measures a self-similarity of said rhythm and/or melody.
Clause 17. A system for representing a biological process via non-speech audio comprising a computational unit configured for:
   (a) extracting a sequence of time-related biological events from the biological process; and
   (b) transforming said sequence of time-related biological events into rhythm and/or melody representative of said time-related biological events thereby representing the biological process via non-speech audio.

## Claims

1. A computer-implemented method of representing a biological process via non-speech audio, comprising, by a computational unit:
receiving a video or time-lapse capture of the biological process;
identifying a sequence of time-related biological events in the video or time-lapse capture; and
transforming the sequence of time-related biological events into an audio track by mapping each biological event to a respective sound element and triggering each sound element at a time determined by the time of the respective time-related biological event in the video or time-lapse capture, thereby rendering the temporal pattern of the biological events audible.

2. The method of claim 1, further comprising:
determining movement between successive frames of the video or time-lapse capture;
producing a data stream corresponding to the movement; and
mapping the data stream onto parameters of a sound synthesizer to generate a drone.

3. The method of claim 2, wherein determining movement between successive frames comprises calculating an average pixel difference between two consecutive frames.

4. The method of claim 2, wherein determining movement between successive frames comprises calculating an optical flow between successive frames, the method further comprising:
separating the optical flow into a plurality of directional data streams, each data stream corresponding to pixel movement in a respective direction; and
mapping the directional data streams onto the parameters of the sound synthesizer to generate the drone, thereby spatializing the drone such that a left or right channel is more or less active based on the amount of optical flow to the left or right.

5. The method of claim 4, wherein a similarity between the directional data streams is mapped to the parameters of the sound synthesizer such that the drone is rendered more distorted when the similarity is lower.

6. The method of claim 1, wherein identifying the sequence of time-related biological events comprises using image recognition software to tag each identified biological event with a time stamp and an identifier.

7. The method of claim 6, wherein the image recognition software comprises a convolutional neural network.

8. The method of any one of claims 1 to 7, wherein each sound element is a note of a musical scale, the biological events being mapped to different notes of the musical scale.

9. The method of claim 8, wherein the frequencies of the notes of the musical scale are determined by the relative spacing in time of the biological events.

10. The method of any one of claims 1-9, further comprising applying time compression or time expansion to the sequence of time-related biological events.

11. The method of any one of claims 1-10, further comprising analysing the audio track by a signal-processing algorithm that measures a self-similarity and/or a rhythmic periodicity of the audio track.

12. The method of claim 11, further comprising outputting, on the basis of said analysing, a determination of whether the biological process is normal or abnormal.

13. A system for representing a biological process via non-speech audio, the system comprising a computational unit configured to carry out the method of any one of claims 1 to 12.

14. The method of any one of claims 1-12, or the system of claim 13, wherein the biological process comprises disease progression, tumor growth, organ development, brain activity, heartbeat patterns, blood pressure changes, respiratory system changes, or embryonic development.

15. The method of any one of claims 1-12, or the system of claim 13, wherein the time-related biological events comprise cell division events.
